# EUROPEAN PATENT SPECIFICATION

(11) **EP 4 255 345 B1**
(45) Date of publication and mention of the grant of the patent: **18.02.2026**
(21) Application number: 21823577.8
(22) Date of filing: 06.12.2021
(51) Int. Cl.: A61F 2/00

(54) **DATA TRANSFER METHOD FOR A PROSTHESIS OR ORTHOSIS AND SYSTEM THEREOF**
DATENÜBERTRAGUNGSVERFAHREN FÜR EINE PROTHESE ODER ORTHESE UND SYSTEM DAFÜR
PROCÉDÉ DE TRANSFERT DE DONNÉES POUR PROTHÈSE OU ORTHÈSE ET SYSTÈME ASSOCIÉ

(30) Priority: 04.12.2020 NL 2027046
(43) Date of publication of application: 11.10.2023
(73) Proprietor: Axiles Bionics BV, 1130 Brussel (BE)
(72) Inventor: CHERELLE, Pierre Philippe, 1472 VIEUX-GENAPPE (BE); CHERELLE, Claire Yvette, 1070 ANDERLECHT (BE); MARULANDA, Felipe Gomez, 1040 ETTERBEEK (BE)
(74) Representative: Arnold & Siedsma
(86) International application number: PCT/EP2021/084425
(87) International publication number: WO 2022/117892

(56) References cited:
- WO-A1-2008/092183
- WO-A1-2009/097563
- WO-A2-2016/009308

## Description

### FIELD OF INVENTION

The field of the invention relates to data transfer methods for prostheses or orthoses, data treatment methods for prostheses or orthoses, systems comprising prostheses or orthoses, energy-charging devices for prostheses or orthoses. Particular embodiments relate to matters pertaining to prostheses or orthoses for functionally assisting, enhancing, and/or replacing a limb of a human or animal subject or for augmenting a body or a part of a body of a human or animal subject.

### BACKGROUND

In existing prostheses or orthoses, collecting data using sensors related to the operation of the prosthesis or orthosis is known to be able to obtain feedback during use. The collected data can be taken into account in order to be able to control more accurately the operation of the prosthesis or orthosis. This is especially the case with prostheses or orthoses including actuators such as motors, or dampers. Indeed, body motion involves the coordination of various interrelated and complex translations and rotations of different body parts. For the user to have a more comfortable experience during use, closer to a natural body motion, monitoring and collecting data is necessary. After collecting data, processing of said data to actively or passively control the different components of the prosthesis or orthosis allows to emulate motions of the corresponding body part. However, the active or passive control of prostheses or orthoses still depends on a predetermined set of configuration and/or behavioral data associated to the components of the prosthesis or orthosis, and has limited robustness and stability.

In prior art solutions, to address the above mentioned problems, maintenance or servicing can be performed personally by an operator. However, such maintenance or servicing can be costly, time-consuming, and cannot be performed frequently with ease.

For example, WO2016009308 describes a prosthesis or orthosis comprising a movement controlling mechanism (MCM) comprising a first MCM part, a second MCM part and one or more intermediate elements and biasing means which, in a contacting mode of operation of the MCM, bias the intermediate elements against a MCM part, while, on the one hand, when a relative torque or force is applied in a blocking sense transmission of torque is allowed and, on the other hand, when a torque or force is applied in the opposite sense non-blocking relative movement is allowed. More in particular a bionic foot is described with a loadcell which allows a force measurement and the elongation of the PO springs is measured with a linear potentiometer. To measure the position of the lever arm, and the leg with respect to the foot, two absolute magnetic encoders are used. As a result of this, the resulting torque at the ankle can be calculated. To detect the important triggers during the stance phase (IC, FF, HO, TO), two Force Sensing Resistors (FSR) are placed on the foot sole: one at the heel and one at the toes. These triggers will be used to control the motor and to unlock locking mechanism 1. A current sensor is also used to measure the current sent to the motor. This information serves essentially in the low level control of the device. WO2009097563 describes that neural protheses may consist of combinations of both external and implanted components.

### SUMMARY

The object of embodiments of the invention is to provide a data transfer method for a prosthesis or orthosis allowing improving the comfort of the prosthesis or orthosis for a user, while relieving the need for a complex and time-intensive maintenance or servicing.

According to a first aspect of the invention, there is provided a data transfer method for a prosthesis or orthosis. The prosthesis or orthosis includes a data storing means and a processing means. The method comprises: obtaining data related to an operation of the prosthesis or orthosis and/or to an environment of the prosthesis or orthosis; storing the obtained data and/or processed data based on the obtained data in the data storing means; transferring, using a communication module connected to the prosthesis or orthosis, at least part of the stored data and/or processed data based on the stored data, to a remote server.

By collecting and storing data related to the operation and/or the environment of the prosthesis or orthosis, relevant data can be gathered and can be used for improving the use of the prosthesis or orthosis. The stored data may be raw data which was directly obtained, e.g. by a sensor, or processed data based on the directly obtained data. Data related to the operation of the prosthesis or orthosis may be data such as logs of control data used during use of the prosthesis or orthosis, inputs to the processor of the prosthesis or orthosis, physical characteristics of different components composing the prosthesis or orthosis, algorithms used for the operation of the prosthesis or orthosis, software data for the processor, or sensor data of sensor means that may be comprised in the prosthesis or orthosis, said sensor means pertaining to the inner working of the prosthesis or orthosis. Data related to the environment of the prosthesis or orthosis may be sensor data of sensor means that may be comprised in the prosthesis or orthosis, said sensor means pertaining to the surroundings of the prosthesis or orthosis. The data related to the environment may relate to parts of the surroundings in which the wearer of the orthosis or prosthesis is present or may relate to parts of the wearer, such as data about a part of the body of the wearer. In an embodiment, data related to the environment may be pertaining to a signal from a neural sensor implanted in the user of the prosthesis or orthosis. Such signal may include motion commands for the prosthesis or orthosis.

At least part of the stored data may be then transferred to the remote server. Alternatively of additionally, processed data based on the stored data may be transferred to the remote server. The data may be processed by the processing means of the prosthesis or orthosis and/or a processing means of the communication module. By transferring data to the remote server, data can be accumulated at the level of the remote server automatically without the need for an intervention by the operator. This data received at the remote server can be used for improving the comfort of the prosthesis or orthosis for a user. Indeed, data will be received at the remote server and will allow making adjustments for each individual wearing such prosthesis or orthosis. Therefore, at least part of the maintenance or servicing can be performed with relative ease and in a time-efficient manner.

The data is transferred to the remote server using the communication module. The communication module may be part of the prosthesis or orthosis, may be an independent unit, and/or may be part of another device separate from the prosthesis or orthosis. The communication module may interface with the data storing means of the prosthesis or orthosis. The interfacing may be achieved wirelessly or in a wired manner. The communication module and the remote server may communicate wirelessly or in a wired manner.

It is to be noted that the processing of data stored in the data storing means may be performed directly after the data is stored or at an ulterior time. Also, the transferring of data to the remote server may be performed directly after the data is stored, after the processing of the stored data, or at an ulterior time.

In the context of the invention the term orthosis is used to refer both to a device used to assist a person with a limb pathology, as well as to a device that augments the performance of an able-bodied wearer. The latter is also called an exoskeleton. Thus, when referring to an orthosis in the context of this application, this could be an orthosis used to assist a person with a limb pathology as well as an exoskeleton.

In the context of the invention the term "remote server" can refer to a single server or a group of servers, e.g. a distributed set of servers, such as an edge cloud.

According to a preferred embodiment, the method further comprises receiving, using the communication module, configuration and/or behavioral data from the remote server. The configuration and/or behavioral data may be obtained based on the transferred data.

In this manner, configuration and/or behavioral data can be transferred to the prosthesis or orthosis without the need for a personal intervention by the operator. The configuration and/or behavioral data influences the way the prosthesis or orthosis operates and/or behaves. Thus the configuration and/or behavioral data may be optimized for each individual based on the data transferred to the remote server. This further improves the time-efficiency of the maintenance or servicing. The configuration data may comprise control data for the operation of the prosthesis or orthosis. Typically, the configuration data may control the internal operation of the prosthesis or orthosis, and the configuration data may be updated by the remote server based on internal information, such as errors, logs, etc. received from the prosthesis or orthosis. The behavioral data may comprise parameters used by the processor during operation of the prosthesis or orthosis. Typically, the behavioral data will determine the external operation of the prosthesis or orthosis, i.e. how the prosthesis or orthosis behaves with respect to the actions or activity the user is performing or in response to environmental properties (e.g. type of grounds, stairs, slopes, information about the person or about a body part of the person wearing the prosthesis or orthosis, etc.). The behavioral data may comprise software code, such as a control model for controlling the operation of a force, resistance or motion generating device for influencing the mechanical behavior and/or movement of a component of the orthosis or prosthesis. By being able to alter configuration and/or behavioral data remotely, the configuration and/or behavioral data can be updated in an improved manner. Although some processing of the collected data may be done by processing means of the prosthesis or orthosis and/or by processing means of the communication module, the remote server may perform further processing, wherein the remote server may further take into account data received from a plurality of prostheses or orthoses. Thus, updated configuration and/or behavioral data may be determined using the (optionally processed) data received by the remote server from a plurality of prostheses or orthoses. It is noted that some configuration and/or behavioral data may be adapted in the same way for multiple prostheses or orthoses whilst other configuration and/or behavioral data may be adapted specifically for a particular individual. Stated differently, embodiments of the invention allow to combine edge processing (by processing means of the prosthesis or orthosis and/or by processing means of the communication module) with cloud processing. Thus, the alteration of the configuration and/or behavioral data may be performed more accurately, adapted to the needs of every individual, and more frequently.

According to an exemplary embodiment, the orthosis or prosthesis comprises a force, resistance or motion generating device, such as an actuator or a damper, for influencing the mechanical behavior and/or movement of a component of the orthosis or prosthesis, and a control module configured to control the force, resistance or motion generating device according to a control model. The configuration and/or behavioral data received from the remote server may be used to modify the control model.

In this way, the operation of the prosthesis or orthosis may be altered remotely by using data transfer. The actuator may be pneumatic, piezo-electric, electric, hydraulic, magnetic, mechanical. In some embodiments the altering may be done "online", e.g. as soon as the data is available and in other embodiments the altering may be done "offline", e.g. the data may be stored first and used later. Also, combinations are possible, e.g. some alterations could be done "online" and others "offline". The damper may be active or passive. In a prosthesis or orthosis comprising a control module and a force, resistance or motion generating device configured for influencing the mechanical behavior and/or movement of components of the prosthesis or orthosis, different parameters may be considered which serve to quantify said mechanical impact.

The control model may receive one or more inputs and may use one or more weight parameters to generate one or more outputs based on the one or more inputs. The control model may define a control policy, e.g. as known in the technical domain of artificial intelligence. For example, the one or more inputs may comprise outputs of one or more sensors, and/or processed data based on the outputs of one or more sensors, and/or other data inputs. For example, the sensor data may further be processed in accordance with a detection algorithm to determine one or more operation categories of the prosthesis or orthosis, e.g. an activity mode. These one or more operation categories may then be input in the control model. The control model may use one or more weight parameters associated with the different inputs. The one or more outputs of the control model allow the control module to actively control the mechanical impact of the actuators or dampers of the prosthesis or orthosis. These weight parameters may be used for adaptive control and different sets of weights can be defined based on the activity mode of the prosthesis or orthosis and based on the movement achieved by the prosthesis or orthosis.

Parameters such as the weight parameters mentioned above may be integrated in control models based on algorithms. Indeed, interpretation of sensor data values and other inputs of the control module may be made via control models. The control models may be physical models which use physics and data inputs to infer an actual state of the prosthesis or orthosis, or may be self-learning models which infer an actual state of the prosthesis or orthosis based on assimilated patterns of inputs.

The configuration and/or behavioral data sent to the communication module may include the parameters mentioned above related to the mechanical behavior and/or movement of a component of the orthosis or prosthesis. However, the skilled person will understand that the configuration and/or behavior data is not limited to those parameters and may comprise data relevant to electronic components, or electronic elements included in components involved in the operation of the prosthesis or orthosis, such as for battery management, thermal management, data storage management, clock, ... In an embodiment, the configuration and/or behavioral data may define one or more new sources of inputs for a control model, or the configuration and/or behavioral data may include a new control model itself.

According to an exemplary embodiment, the prosthesis or orthosis further comprises at least one sensor means. The step of obtaining data related to the operation of the prosthesis or orthosis and/or to the environment of the prosthesis or orthosis comprises obtaining sensor data from the at least one sensor means.

In this way, actual use data of the operation of the prosthesis or orthosis and/or of the environment of the prosthesis or orthosis can be gathered. Determining the configuration and/or behavioral data based on sensing data allows for a more accurate adjustment of the comfort of the prosthesis or orthosis for the user. It allows the configuration and/or behavioral data to be adapted for each individual user.

Although some processing of the collected sensor data may be done by processing means of the prosthesis or orthosis and/or by processing means of the communication module, the remote server may perform further processing, wherein the remote server may further take into account sensor data received from a plurality of prostheses or orthoses. Thus, updated configuration and/or behavioral data may be determined using the (optionally processed) sensor data received from a plurality of prostheses or orthoses. It is noted that some configuration and/or behavioral data may be adapted in the same way for multiple prostheses or orthoses whilst other configuration and/or behavioral data may be adapted specifically for a particular individual.

According to a preferred embodiment, the at least one sensor means comprises any one or any combination of: a neural sensor, an angle-sensing means, an accelerometer, a Hall sensor, a force sensor, a magnetometer, a pressure sensor, a torque sensor, a temperature sensor, an energy metering means, a current sensor, a voltage sensor, a humidity sensor, a sonar sensor, an EMG sensor, a barometric sensor, a grid of pressure sensor, an EEG sensor, a RFID sensor, a geo-localization sensor.

In this manner, various types of data may be collected relative to the operation and/or environment of the prosthesis or orthosis. Depending on the sensor means chosen, different algorithm models using the sensor data may be developed in order to infer a state of the prosthesis or orthosis.

It is noted that the obtained data may also comprise other data than sensor data, such as data received from an implant in the body of the wearer or data received from another device worn by the wearer of the prosthesis or orthosis.

According to an exemplary embodiment, the control module controls the force, resistance or motion generating device based on sensor data of the at least one sensor means. More in particular, the control model may receive one or more inputs from one or more sensors of the at least one sensor, and may generate an output based on said one or more sensor inputs, optionally in combination with other inputs.

In this way, actual use of the prosthesis or orthosis may be taken into account directly to adapt in an accurate manner the movements of the prosthesis or orthosis via the force, resistance or motion generating device. In addition, the control model itself and/or one or more parameters used therein may be part of the configuration and/or behavioral data which may be updated and adapted to an individual user based on cloud processing of data received from one or more orthoses or prostheses.

According to a preferred embodiment, the at least one sensor means comprises multiple sensors. One or more sensors of said multiple sensors are selected to provide an input of the control model based on the configuration and/or behavioral data.

In this manner, the most appropriate sensor or set of sensors may be selected in order to regulate in an improved manner the mechanical behavior and/or movements of the prosthesis or orthosis via the force, resistance or motion generating device. Thus, depending on the configuration and/or behavioral data, different sets of one or more sensors may be used as an input of the control model. Additionally or alternatively, different set of one ore more sensors may be used as an input of the control model depending on environmental data.

According to a preferred embodiment, the at least one sensor means comprises a first set of sensor means and a second set of sensor means. Depending on an activity mode of the prosthesis or orthosis in use, first processed data is determined based on sensor data from the first set of sensor means, and/or second processed data is determined based on sensor data from the second set of sensor means, and said first and/or second processed data is transferred to the remote server. The first set of sensor means may be different from the second set of sensor means, but it is noted that the first and the second set of sensor means may have one or more sensor means in common. Further, the first way of processing to obtain the first processed data may be different from the second way of processing to obtain the second processes data.

In this manner, an activity mode of the prosthesis or orthosis is taken into account to determine which kind of sensor data and which kind of processing is to be done. In that way the amount of sensing and processing at the edge may be limited and optimized in function of the activity mode.

By activity mode, it is meant the type of motion implemented thanks to or via the prosthesis or orthosis by the user. An activity mode may be defined based on a trajectory of motion, such as a gait pattern; a time duration of motion; and/or on various data sensed during the execution of the motion. An activity mode may also be related to an activity level, *i.e.* how active a user is. An activity level may be determined e.g. based on sensed motion. For example, activity modes could be defined as: standing, walking below a certain speed, walking above a certain speed, running, jumping, sitting, etc.

According to an exemplary embodiment, the at least one sensor means comprises a first set of sensor means and a second set of sensor means. Depending on a current activity mode and/or current environmental data and/or current location data of the prosthesis or orthosis, first processed data is determined based on sensor data from the first set of sensor means, and/or second processed data is determined based on sensor data from the second set of sensor means, and said first and/or second processed data is transferred to the remote server. The first set of sensor means may be different from the second set of sensor means, but it is noted that the first and the second set of sensor means may have one or more sensor means in common. Further, the first way of processing to obtain the first processed data may be different from the second way of processing to obtain the second processes data. In this manner, the activity and/or the environment and/or the location of the user may be taken into account to determine one or more appropriate sets of sensor means which will typically be subsets of all available sensor means. In this manner processing power may be saved and the processing may be done in a more efficient manner, either locally by the prosthesis or orthoses or by the communication module, or remotely by the remote server.

In order to determine the location data, optionally the orthoses or prosthesis may be provided with a GPS module. Alternatively, location data may be received from a remote device.

In another embodiment, the at least one sensor means comprises a first set of sensor means and a second set of sensor means, and first processed data is determined based on sensor data from the first set of sensor means and associated with a first priority, and second processed data is determined based on sensor data from the second set of sensor means and associated with a second priority, wherein the timing of the transferring of the first and/or second processed data to the remote server is based on the associated priority level.

In that manner, processed data with a high priority level may be sent to the remote server e.g. also when the available bandwidth is limited whilst other processed data with a lower level of priority may be sent only when sufficient bandwidth is available.

According to a preferred embodiment, the method further comprises: processing, by the processing means of the prosthesis or orthosis, data related to the operation of the prosthesis or orthosis and/or to the environment of the prosthesis or orthosis stored in the data storing means, and storing said processed data in the data storing means. The step of transferring data to the remote server comprises transferring, using the communication module, at least part of the processed data to the remote server.

In this manner, the processing burden on the remote server may be lessened and the amount of data transferred to the remote server may be decreased by sending processed data, instead of raw data for example. The processing of data by the processing means may be performed upon storing obtained data from the prosthesis or orthosis or after a delay. The processing of data by the processing means may be performed immediately prior to the transfer of data to the remote server or a substantial amount of time prior to the data transfer to the remote server.

According to a preferred embodiment, the communication module is connected through a wired connection to the prosthesis or orthosis.

In this manner, the connection for transfer of data by the communication module may be more reliable and secured.

According to a preferred embodiment, the communication module is included in a separate device, i.e. a device which is not integrated with the prosthesis or orthosis.

In this manner, transfer of data to the remote server can be performed at select times, *i.e.* when the communication module of the separate device is connected to the prosthesis or orthosis, and safety of the data transfer of the prosthesis or orthosis can be improved. In an embodiment, the communication module may be a part of a device having multiple functions. In another embodiment, the communication module may be an independent unit only, *e.g.* an antenna unit, configured for communication with the remote server. Depending on embodiments, the separate device may be a portable battery device, a mobile communication device, or a device intended for home use.

In an embodiment, there is also provided a data transfer method for the separate device comprising: obtaining data related to an operation of the separate device and/or to an environment of the separate device; transferring, using the communication module, at least part of the obtained data and/or processed data based on the obtained data, to the remote server.

By collecting data related to the operation and/or the environment of the separate, relevant data can be gathered and can be used for improving the use of the separate device, and more specifically the communication module. The stored data may be raw data which was directly obtained, or processed data based on the directly obtained data. Data related to the operation of the separate device may be data such as logs of control data used during use of the separate device, inputs to the processor of the separate device, and more specifically to the communication module, physical characteristics of different components composing the separate device, algorithms used for the operation of the separate device, software data for the separate device, or sensor data of sensor means that may be comprised in the separate device, said sensor means pertaining to the inner working of the separate device. Data related to the environment of the separate device may be sensor data of sensor means that may be comprised in the separate device, said sensor means pertaining to the surroundings of the separate device.

At least part of the obtained data and/or processed data may be then transferred to the remote server. By transferring data to the remote server, data can be accumulated at the level of the remote server automatically without the need for an intervention by the operator. This data received at the remote server can be used for improving the capacities of the separate device, and more specifically of the communication module. Indeed, data will be received at the remote server and will allow making adjustments for said separate device. The adjustments may relate to *e.g.* send the data optimally, charging the prosthesis or orthosis in a faster manner, optimize the processing time of the data by the prosthesis or orthosis or the separate device.

According to an exemplary embodiment, the separate device is an energy charging device and the prosthesis or orthosis comprises an energy storage means. The method further comprises charging the energy storage means via the energy charging device.

In this way, communication with the remote server can occur at the same time as the electrical charging of the prosthesis or orthosis which is more time efficient. The energy charging device may be a stationary charging device or a portable energy device. Additionally, the energy charging device may be charging the prosthesis or orthosis in a wired manner or wirelessly, *e.g*. inductively.

In an embodiment, the energy charging device may be a stationary charging device plugged to a power grid and the communication module uses a Wifi connection or an Ethernet connection to communicate with the remote server; the prosthesis or orthosis may be plugged to the stationary charging device during a time when it is not in use, e.g. during night time for charging and transferring data to the remote server simultaneously. In another embodiment, the energy charging device may be a portable charging device, such as a portable battery device, connected electrically to the prosthesis or orthosis in a wired manner and the communication module may be connected wirelessly to the prosthesis or orthosis, *e.g*. using Bluetooth, and communicating with the remote server wirelessly, *e.g*. Wifi, 4G, 5G. In yet another embodiment, the energy charging device may be configured for charging the prosthesis or orthosis in an inductive manner and the user may charge his associated prosthesis or orthosis inductively while wearing it. The skilled person will understand that using currently available technologies, various embodiments of the energy charging device can be implemented which are configured for both charging and communicating.

According to a preferred embodiment, the separate device comprises a second data storing means connected to the communication module. The step of transferring data to the remote server comprises a first step of transferring at least part of the stored data and/or processed data based on the stored data to the second data storing means, followed by a second step of transferring the at least part of the data stored in the second data storing means and/or processed data based thereon to the remote server.

In this manner, the amount of data stored in the data storing means can be transferred regularly and the amount of data accumulated in the data storing means may be thusly regulated, which allows lessening the weight and energy impacts of having extra components part of the prosthesis or orthosis. For example, the amount of data that may be stored locally in the data storing means is equivalent to a number of days of data, such as one or two days of data; and the amount of data that may be stored in the second data storing means is equivalent to a larger number of days of data, e.g. one or two weeks. In an embodiment, the transferring of data from the data storing means to the second data storing is followed by a delay before the transferring of data to the remote server. For example, the transferring may take place when the separate device is connected to a power grid. When transferring data to the remote server, the data transferred to the remote server may originate from the data storing means and/or the second data storing means.

According to an exemplary embodiment, the separate device comprises a second processing means configured for processing the data stored in the second data storing means. The second processing means of the separate device is configured for storing the processed data in the second data storing means.

In this way, the processing burden on the remote server and/or in the processing means of the prosthesis or orthosis may be lessened and the amount of data transferred to the remote server may be decreased by sending processed data, instead of raw data for example. The processing of data by the second processing means may be performed upon storing obtained data from the prosthesis or orthosis or after a delay. The processing of data by the second processing means may be performed just prior to transferring data to the remote server or a substantial amount of time prior to the data transfer to the remote server.

Thus, preprocessing and/or further processing may be performed in the separate device, and the preprocessed or further processed may be sent to the remote server at a suitable time, e.g. when the available bandwidth is sufficiently high. Stated differently, a certain amount of edge computing may take place on the separate device.

In some exemplary embodiments, multiple separate devices may be associated with a prosthesis or orthoses. For example, a first separate device with an implemented charging functionality but with no or limited processing power, and a second separate device without charging functionality but with more processing power than that of the first separate device. In such an embodiment, the second separate device may be configured to perform e.g. automatic learning based on the data received from the prosthesis or orthoses, as this type of processing typically requires a lot of computation power.

According to a preferred embodiment, the first step of transferring to the second data storing means is performed at more frequent time intervals than the second step of transferring to the remote server.

In this manner, the second data storing means may serve to manage the amount of data stored in the data storing means. Typically, the second data storing means may be larger than the data storing means; so the amount of data to be stored in the data storing means may be kept below a certain value in order to minimize the impact of the data storing means on the use of the prosthesis or orthosis.

According to an exemplary embodiment, the first step of transferring to the second data storing means is performed during a substantially inactive state of the prosthesis or orthosis.

In this way, the transferring of data may be achieved in a stable state of the prosthesis or orthosis. By substantially inactive state, it is meant a state of the prosthesis or orthosis when there is no motion implemented during a predetermined amount of time, e.g. at least 3-5 minutes. In an embodiment, the substantially inactive state may correspond to an energy sleeping state of the prosthesis or orthosis. In another embodiment, the substantially inactive state may correspond to a state after a period of time during which the prosthesis or orthosis attached to the user has been immobile.

According to an exemplary embodiment, the communication module is included in the prosthesis or orthosis.

In this way, the transferring of data to the remote server may be performed with more flexibility relative to time. In an embodiment, the communication module may be transferring data wirelessly and the data may be transferred upon detecting an available transfer data rate of the communication module being above a preset threshold. In another embodiment, the communication module may be transferring data upon detecting an amount of data stored in the data storing means being above a preset threshold.

It is further noted that there may be separate device with a communication module as described above, in addition to a communication module in the prosthesis or orthosis.

According to an exemplary embodiment, the step of obtaining data comprises collecting data generated during use of the prosthesis or orthosis.

In this way, the data generated during use may be stored directly in the data storing means.

According to a preferred embodiment, the method further comprises obtaining further data related to an operation of the prosthesis or orthosis and/or to an environment of the prosthesis or orthosis and determining whether or not the further obtained data is to be stored based on data previously stored in the data storing means.

In this manner, a filtering on the obtained data can be implemented and the stored data may be better managed with a higher relevancy of the stored data. In an embodiment, the step of determining may be based on whether or not similar data is already present in the data storing means. For example, if the wearer of the prosthesis or orthosis has been doing the same activity for a certain amount of time, e.g. walking, it may be decided to no longer store the further obtained data as long as the activity is not changed. In that manner the data amount stored per activity mode may remain below a preset threshold. In another embodiment, the obtained data may be associated with a priority level and only further obtained data above a certain priority level are selected to be stored.

According to an exemplary embodiment, the transferring of data to the remote server is performed periodically, preferably daily.

In this way, the obtained data stored can be managed and transferred in a reliable manner.

According to an exemplary embodiment, an identification of the prosthesis or orthosis or of the communication module is added to the data which is transferred to the remote server.

In that manner, when multiple prostheses or orthoses communicate with the remote server, a distinction can be made between different prostheses or orthoses. Also, the determining of configuration and/or behavioral data for a specific prosthesis or orthosis can be done based on data received from that prosthesis or orthosis, optionally also taking into account data received from other prostheses or orthoses.

According to an exemplary embodiment, the prosthesis or orthosis referred to above corresponds with a first prosthesis or orthosis, and the method further comprises the following steps performed by a second prosthesis or orthosis: obtaining second data related to an operation of the second prosthesis or orthosis and/or to an environment of the second prosthesis or orthosis; storing the obtained second data and/or processed data based on the obtained second data; and transferring, using a communication module connected to the second prosthesis or orthosis, at least part of the stored second data and/or processed second data based on the stored second data, to the remote server. Further, the method may comprise the following steps performed by the remote server:
- determining of first and second configuration and/or behavioral data for the first and second prosthesis or orthosis, respectively, based on the transferred data received from the first and second prosthesis or orthosis;
- transmitting the determined first and second configuration and/or behavioral data to the communication module associated with the first and second prosthesis or orthosis, respectively.

More in particular, determining of the first configuration and/or behavioral data for the first prosthesis or orthosis, may be based on the transferred data received from the first prosthesis or orthosis, but optionally also based on data received from the second prosthesis or orthosis. Similarly, determining of the second configuration and/or behavioral data for the second prosthesis or orthosis, may be based on the transferred data received from the second prosthesis or orthosis, but optionally also based on data received from the first prosthesis or orthosis. In that way the configuration and/or behavioral data may be personalized for a particular user whilst at the same time taking into account problems encountered by another user.

The exemplary embodiment is described here for two orthoses or prostheses, but the skilled person understands that a similar method may be used using data from more than two orthoses or prostheses. Further, users may be classified in groups on the remote server, e.g. based on the activity mode and/or environmental data and/or location data, and the further processing by the remote server may be done using data received from a group of users classified in the same class.

According to an exemplary embodiment, the communication module is configured to communicate with the remote server in a secure manner. For example, the communication module may be configured to perform a handshaking with the remote server before any data is exchanged to guarantee data security. Optionally encryption keys or other encryption methods may be used. In a similar manner, if a separate device is used as described above, any data exchanged between the data storing means of the orthoses or prosthesis and the second data storing means of the separate device, and/or between the second data storing means and the remote server, are preferably performed in a secure manner.

The skilled person will understand that the hereinabove described technical considerations and advantages for data transfer method embodiments also apply to the below described corresponding data treatment method embodiments, *mutatis mutandis.*

According to a second aspect of the invention, there is provided a data treatment method for a prosthesis or orthosis. The method performed in a remote server comprises: receiving data from a communication module, said data comprising data related to an operation of the prosthesis or orthosis and/or to an environment of the prosthesis or orthosis and/or processed data based on said data related to the operation of the prosthesis or orthosis and/or to the environment of the prosthesis or orthosis; determining configuration and/or behavioral data for operating the prosthesis or orthosis based on the received data; sending the configuration and/or behavioral data to the communication module.

By sending back configuration and behavioral data to the prosthesis or orthosis, a present operational behavior of the prosthesis or orthosis may be altered in order to improve said operational behavior of the prosthesis or orthosis. The improvement may be in terms of comfort of the user using the prosthesis or orthosis, energy management, thermal management, error management, etc.

According to an exemplary embodiment, the prosthesis or orthosis corresponds with a first prosthesis or orthosis, and the method further comprises: receiving second data from a communication module associated with a second prosthesis or orthosis, said second data comprising data related to an operation of the second prosthesis or orthosis and/or to an environment of the prosthesis or orthosis and/or processed data based on said data; determining second configuration and/or behavioral data for the second prosthesis or orthosis, based on the received second data, and further based on the data received from the first prosthesis or orthosis; sending the second configuration and/or behavioral data to the communication module associated with the second prosthesis or orthosis.

In that way the configuration and/or behavioral data may be personalized for a particular user whilst at the same time taking into account problems encountered by another user.

The exemplary embodiment is described here for two orthoses or prostheses, but the skilled person understands that a similar method may be used, using data from more than two orthoses or prostheses. Further, users may be classified in groups on the remote server, e.g. based on the activity mode and/or environmental data and/or location data, and the further processing by the remote server may be done using data received from a group of users classified in the same class.

The skilled person will understand that the hereinabove described technical considerations and advantages for data transfer method embodiments and data treatment method embodiments also apply to the below described corresponding system embodiments, *mutatis mutandis.*

According to a third aspect of the invention, there is provided a system comprising a prosthesis or orthosis and a communication module. The system may be for use in a data transfer method as described above. The prosthesis or orthosis comprises a data storing means, and a processing means. The data storing means is configured for storing data related to an operation of the prosthesis or orthosis and/or to an environment of the prosthesis or orthosis. The communication module comprises a communication interface configured for transferring at least part of the stored data and/or processed data based thereon, to a remote server.

Preferred embodiments of the system are described in claims 23-36.

The skilled person will understand that the hereinabove described technical considerations and advantages for data transfer method embodiments, data treatment method embodiments, and system embodiments also apply to the below described corresponding energy charging device embodiments, *mutatis mutandis.*

According to a fourth aspect of the invention, there is provided an energy charging device for a prosthesis or orthosis comprising an energy storage means. The energy charging device comprises: an electrical interface configured for cooperating with a corresponding electrical interface of the prosthesis or orthosis such that the energy storage means of the orthosis or prosthesis is being charged; and a communication module configured for receiving data stored in a data storing means of the prosthesis or orthosis, and for communicating with a remote server.

According to an exemplary embodiment, the energy charging device comprises a processing means configured for processing the data received from the prosthesis or orthosis.

This processing means may correspond with any one of the embodiments of the second processing means described above.

According to an exemplary embodiment, the energy charging device comprises a second electrical interface configured to be connected to a power grid.

This second electrical interface may be used to directly provide power from the grid to the prosthesis or orthosis, optionally via a converter means to covert the grid power to a suitable power level for feeding the prosthesis or orthosis. Alternatively or in addition, the energy charging device may comprise a battery and the second electrical interface may be used to charge the battery. The battery may then be used to provide power to the prosthesis or orthosis.

The skilled person will understand that the hereinabove described technical considerations and advantages for data transfer method embodiments, data treatment method embodiments, system embodiments, and energy charging device embodiments also apply to the below described corresponding data transfer methods for medical devices embodiments, *mutatis mutandis.*

According to a fifth aspect of the invention, there is provided a data transfer method for a medical device configured for being attached to a human body. The medical device comprises a data storing means and a processing means. The method performed by the processing means comprises: obtaining data related to an operation of the medical device and/or to an environment of the medical device; storing the obtained data in the data storing means; transferring, using a communication module connected to the medical device, at least part of the stored data and/or processed data based on the stored data, to a remote server. Preferably, the communication module is included in a separate device, and the separate device may have any one of the features described above. For example, the separate device may be an energy charging device as described above.

According to a further aspect of the invention, there is provided a computer program comprising computer-executable instructions to perform the method, when the program is run on a computer or, according to any one of the steps of any one of the embodiments disclosed above.

According to a further aspect of the invention, there is provided a computer device or other hardware device programmed to perform one or more steps of any one of the embodiments of the method disclosed above. According to another aspect there is provided a data storage device encoding a program in machine-readable and machine-executable form to perform one or more steps of any one of the embodiments of the method disclosed above.

### BRIEF DESCRIPTION OF THE FIGURES

This and other aspects of the present invention will now be described in more detail, with reference to the appended drawings showing a currently preferred embodiment. Like numbers refer to like features throughout the drawings.
Figure 1 depicts a flow chart of exemplary embodiments of a data transfer method and a data treatment method for a prosthesis or orthosis;
Figure 2 shows a schematic view of an exemplary embodiment of a system comprising a prosthesis or orthosis communicating with a remote server;
Figure 3 shows an exemplary embodiment of a network system with multiple prostheses or orthoses;
Figure 4 illustrates schematically an exemplary embodiment of an energy charging device and a prosthesis or orthosis;
Figure 5 shows a schematic view of another exemplary embodiment of a control module implemented in a prosthesis or orthosis.

### DESCRIPTION OF EMBODIMENTS

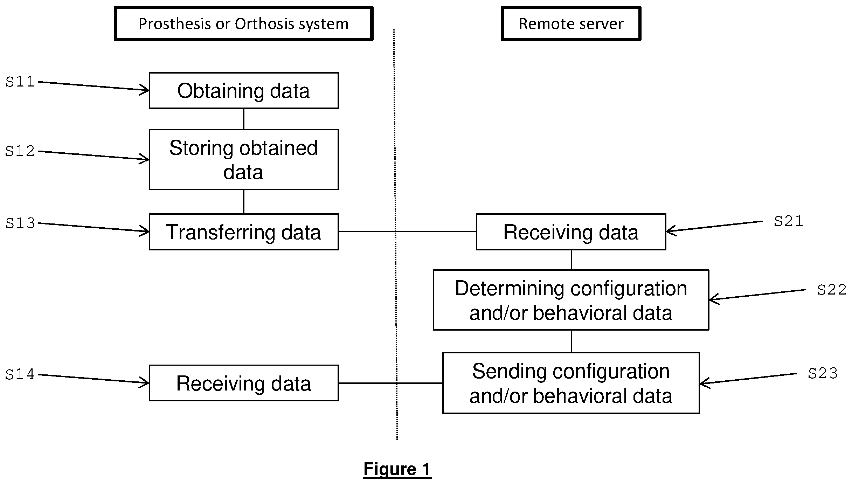

Figure 1 depicts a flow chart of an exemplary embodiment of a data transfer method and an exemplary embodiment of a data treatment method according to the present invention. The data transfer method and the data treatment method are for a prosthesis or orthosis. The prosthesis or orthosis includes a data storing means and a processing means. A communication module connected to the data storing means of the prosthesis or orthosis communicates to a remote server. The prosthesis or orthosis may be configured for functionally assisting, enhancing, and/or replacing a limb of a human or animal subject or for augmenting a body or a part of a body of a human or animal subject. Thus, the term orthosis is used to refer both to a device used to assist a person with a limb pathology, as well as to a device that augments the performance of an able-bodied wearer. Thus, when referring to an orthosis, this could be an orthosis used to assist a person with a limb pathology as well as an exoskeleton.

Prostheses or orthoses may comprise a force, resistance or motion generating device such as actuators and/or dampers for influencing the mechanical behavior and/or movement of a component of the orthosis or prosthesis. In prosthesis or orthosis comprising a control module configured for controlling such force, resistance or motion generating device, different parameters may be considered. For example, parameters may be used in feedback loops as weights for different considered inputs, such as sensor data. A force, resistance or motion generating device, such as an actuator and/or a damper may then be actively or passively controlled based on the feedback loops.

In a first step S11, the method comprises obtaining data related to an operation of the prosthesis or orthosis and/or to an environment of the prosthesis or orthosis. Data related to the operation of the prosthesis or orthosis may be data such as logs of control data used during use of the prosthesis or orthosis, inputs to the processing means of the prosthesis or orthosis, physical characteristics of different parts composing the prosthesis or orthosis, algorithms used for the operation of the prosthesis or orthosis, or sensing data of sensor means that may be comprised in the prosthesis or orthosis, said sensor means pertaining to the operation of the prosthesis or orthosis. Data related to the environment of the prosthesis or orthosis may be sensor data of sensor means that may be comprised in the prosthesis or orthosis, said sensor means pertaining to the surroundings of the prosthesis or orthosis. In an embodiment, data related to the environment may be pertaining to signals from a neural sensor implanted in the user of the prosthesis or orthosis, said signals including e.g. motion commands for the prosthesis or orthosis. Further, the prosthesis or orthosis may also send signals to the neural sensor.

In an embodiment, the prosthesis or orthosis may comprise at least one sensor means, and the step of obtaining data may comprise obtaining sensor data from the at least one sensor means. Depending on embodiments, the at least one sensor means may comprise any one or any combination of: an angle-sensing means, an accelerometer, a Hall sensor, a force sensor, a magnetometer, a pressure sensor, a torque sensor, a temperature sensor, an energy metering means, a current sensor, a voltage sensor, a humidity sensor, a sonar sensor, an EMG sensor, a barometric sensor, a grid of pressure sensor, an EEG sensor, a RFID sensor, a neural sensor, a geo-localization sensor.

Preferably, the first step S11 of obtaining data may be performed during use of the prosthesis or orthosis. However, some data may also be obtained at an ulterior time.

In a second step S12, the method comprises storing the obtained data in the data storing means. The obtained data may be raw or processed data related to the operation of the prosthesis or orthosis and/or to the environment of the prosthesis or orthosis stored in the data storing means. Optionally the stored data may be further processed after step S12 and before step S13. All the obtained data in step S11 may be further processed or only a portion of it. The processing may be performed by the processing means of the prosthesis or orthosis and/or by a second processing means included in a separate device comprising the communication module, as will be further explained below.

The step S12 of storing the obtained data may comprise selecting a portion of the obtained data to be stored based on the stored data in the data storing means. In an embodiment, the selecting of the portion of the obtained data may be based on an amount of data collected corresponding to an activity mode of the prosthesis or orthosis and only data corresponding to activity modes with data amounts below a preset threshold may be stored. By activity mode, it is meant to be the type of motion implemented thanks to or via the prosthesis or orthosis by the user. An activity mode may be defined based on a trajectory of motion, a time duration of motion, and/or on sensor data values sensed during the execution of the motion. In another embodiment, the obtained data may be associated with a priority level and only data above a certain priority level may be selected to be stored. For example, obtained data related to an incident during operation of the prosthesis or orthosis may be associated with a high priority level and selected for being stored.

The communication module may be included in a separate device which comprises a second data storing means connected to the communication module. The step S12 may comprise transferring at least part of the stored data and/or the data processed based on the stored data to the second data storing means. Typically, the amount of data that may be stored in the data storing means of the prosthesis or orthosis is equivalent to a number of days of data; and the amount of data that may be stored in the second data storing means connected to the communication module is equivalent to a larger number of days of data. The transferring from the data storing means to the second data storing means may be performed upon performing step S11 or at an ulterior time. In an embodiment, data from the data storing means may be processed by the processing means prior to being transferred to the second data storing means and/or may be processed by the second processing means.

Typically, the second data storing means may be larger than the data storing means; so the data storing means may be kept below a certain size in order to minimize the impact of the data storing means on the prosthesis or orthosis.

Additionally, the at least one sensor means may comprise a first set of sensor means and a second set of sensor means. The first set of sensor means and the second set of sensor means may be partly composed of the same sensor means or may be composed entirely of different sensor means. The stored data obtained from the first set of sensor means and/or the second set of sensor means may be processed. The processed data may comprises first processed data based on sensor data from the first set of sensor means, and second processed data based on sensor data from the second set of sensor means. Depending whether first processed data or second processed data is considered, the use of the processed data may be different. In an embodiment, data from the first set of sensor means may be processed differently than data from the second set of sensor means. In another embodiment, first processed data and second processed data may originate from different types of sensor means, e.g. environment sensor means, operation sensor means. In yet another embodiment, first processed data may have been processed by the processing means of the prosthesis or orthosis, and second processed data may have been processed by the second processing means.

In a third step S13, the method comprises transferring, using the communication module connected to the prosthesis or orthosis, at least part of the stored data and/or processed data based on the stored data, to the remote server. The step S13 may be performed immediately after performing S12 or may be performed at an ulterior time. The transferring to the remote server may be performed periodically, preferably daily.

The step S13 of transferring to the remote server may comprise a first step of transferring to the second data storing means, said first step may be performed during a substantially inactive state of the prosthesis or orthosis. By substantially inactive state, it is meant a state of the prosthesis or orthosis when there is no motion implemented during a predetermined amount of time. In an embodiment, the substantially inactive state may correspond to a sleep energy state of the prosthesis or orthosis. In another embodiment, the substantially inactive state may correspond to a state after a period of time during which the prosthesis or orthosis attached to the user has been immobile.

In an embodiment, stored obtained data may be processed and the transferring of step S13 may be performed upon processing the stored obtained data. In another embodiment, stored obtained data may be processed and the transferring of step S13 may be performed after a delay after processing the stored obtained data.

The stored data, obtained and/or processed, may be associated with different priority levels depending on a relevancy and/or urgency of the data, and data with a higher priority level may be transferred to the remote server before data with a lower priority level. For example, first processed data and second processed data may be associated with different priority levels.

The prosthesis or orthosis comprises the data storing means, and the communication module may be also connected to the second data storing means. When transferring data to the remote server, the data transferred to the remote server in step S13 may originate from the data storing means, and/or the second data storing means. In an embodiment, data stored in the data storing means may be transferred to the second data storing means, and the step of transferring to the second data storing means may be performed at more frequent time intervals than the step of transferring data from the second data storing means to the remote server.

After step S13 performed in a prosthesis or orthosis system comprising the prosthesis or orthosis and the communication module, the transferred data may be treated in the remote server. The method performed in the remote server comprises a step S21 of receiving data from the communication module, said data comprising data related to the operation of the prosthesis or orthosis and/or to the environment of the prosthesis or orthosis and/or processed data based on said data related to the operation of the prosthesis or orthosis and/or to the environment of the prosthesis or orthosis.

The method further comprises a step S22 of determining configuration and/or behavioral data for operating the prosthesis or orthosis based on the received data. The configuration and/or behavioral data may be determined by processing said received data in various manners. The configuration and/or behavioral data may be related to the operation of various electronic components, or components including electronic elements included in the prosthesis or orthosis.

In an embodiment, the configuration and/or behavioral data are related to the operation of a force, resistance or motion generating device, such as an actuator or a damper, of the prosthesis or orthosis. The actuator or the damper may affect the movement of a component of the prosthesis or orthosis. During use, the actuator or the damper action may be defined based on a set of parameters affecting feedback loops for the control of the actuator or the damper. The configuration and/or behavioral data may comprise new values for at least a portion of the set of parameters in order to obtain a more natural motion of the prosthesis or orthosis. Typically, the behavioral data will determine the external operation of the prosthesis or orthosis, i.e. how the prosthesis or orthosis behaves with respect to the actions or activity the user is performing or in response to environmental properties (e.g. type of grounds, stairs, slopes, information about the person wearing the prosthesis or orthosis, etc.). The behavioral data may comprise software code, such as a control model for controlling the operation of a force, resistance or motion generating device for influencing the mechanical behavior and/or movement of a component of the orthosis or prosthesis.

Note that configuration and/or behavioral data may be obtained without using the received data of step S21. In another embodiment, configuration and/or behavioral data may be obtained further based on historical data received at a time prior to step S21. In yet another embodiment, configuration and/or behavioral data may be obtained without relying on received data.

The method further comprises a step S23 of sending the configuration and/or behavioral data to the communication module.

After step S23 performed in the remote server, the method performed in the prosthesis or orthosis system may further comprise a step S14 of receiving, using the communication module, configuration and/or behavioral data from the remote server.

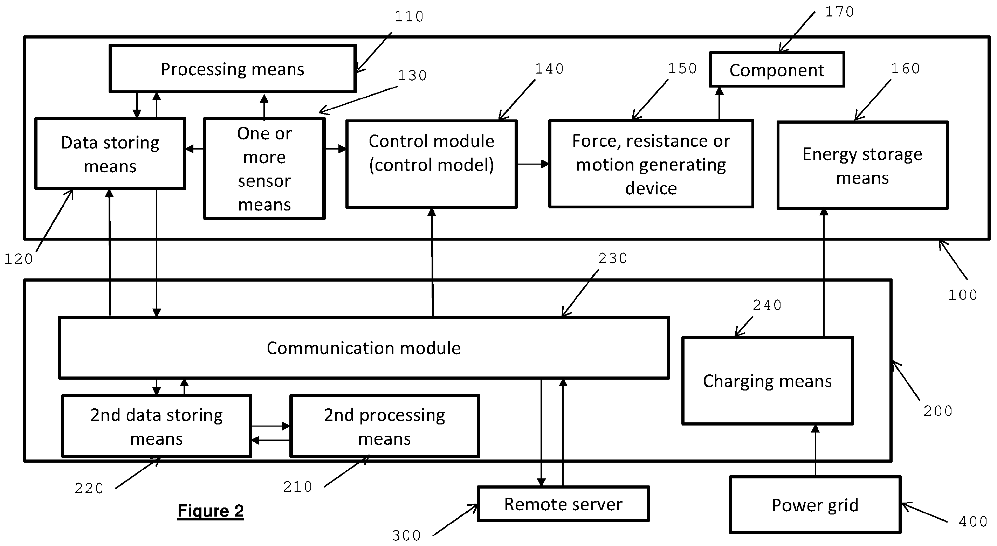

Figure 2 illustrates an exemplary embodiment of a system comprising a prosthesis or orthosis 100 and a separate device 200 comprising a communication module 230. However, in other embodiments the communication module 230 may be included in the prosthesis or orthosis 100.

The prosthesis or orthosis 100 comprises a data storing means 120 and a processing means 110. The data storing means 120 is configured for storing data related to an operation of the prosthesis or orthosis and/or to an environment of the prosthesis or orthosis. The communication module 230 comprises a communication interface configured for transferring at least part of the stored data and/or processed data based thereon, to a remote server 300. The remote server 300 is configured for determining configuration and/or behavioral data based on the transferred data, and the communication module 230 is configured to receive the configuration and/or behavioral data from the remote server 300.

The orthosis or prosthesis 100 may comprises a force, resistance or motion generating device 150, such as an actuator or a damper, for influencing the mechanical behavior and/or movement of a component 170 of the orthosis or prosthesis, and a control module 140 configured to control the force, resistance or motion generating device 150 according to a control model. It is noted that the control module 140 does not have to be a separate module and that is may be integrated with the processing means 110. The configuration and/or behavioral data received from the remote server 300 may be used to adapt the control model used by the control module 140.

The prosthesis or orthosis 100 further comprises at least one sensor means 130 for obtaining sensor data related to the operation of the prosthesis or orthosis and/or to the environment of the prosthesis or orthosis, and the data storing means 120 may store the sensor data and/or processed data based on the sensor data.

The control module 140 is configured to control the force, resistance or motion regulating device 150 based on sensor data of the at least one sensor means 130 in accordance with the control model. Typically, the at least one sensor means 130 comprises multiple sensors, and wherein the control module 140 is configured to select one or more sensors of said multiple sensors 130 as an input of the control model based on the configuration and/or behavioral data.

The at least one sensor means 130 may comprise a first set of sensor means and a second set of sensor means; and the processing means 110 may be configured to determine, depending on an activity mode of the prosthesis or orthosis 100 in use, first processed data based on sensor data from the first set of sensor means, and/or second processed data based on sensor data from the second set of sensor means. In that way the data stored in the data storing means 110**,** and optionally transferred to the remote server 300, may adapted to be suited to the current activity mode. For example, depending on whether an individual is standing or walking or running, it may be desirable to sense different data and/or the process sensed data in a different manner.

Preferably, the communication module 230 is connected through a wired connection to the prosthesis or orthosis 100.

In the illustrated embodiment the separate device 200 is an energy charging device, and the prosthesis or orthosis 100 comprises an energy storage means 160. The energy charging device 200 comprises a charging means 240, e.g. an energy conversion means and/or a battery, which is configured for charging the energy storage means 160.

The separate device 200 comprises a second data storing means 220 connected to the communication module 230, and a second processing means 210 configured for processing data stored in the second data storing means 220, and configured for storing the processed data in the second data storing means 210. In the illustrated embodiment the communication module is included in the separate device 200, but in other embodiments the communication module is included in the prosthesis or orthosis 100. In that case the second processing means 210 may be merged with the processing means 110, and the second data storing means 220 may be merged with the data storing means 120.

Preferably, the processing means 110 and/or the second processing means 210 is configured to add an identification of the prosthesis or orthosis 100 or of the communication module 230/the separate device 200 to the data which is transferred to the remote server 300.

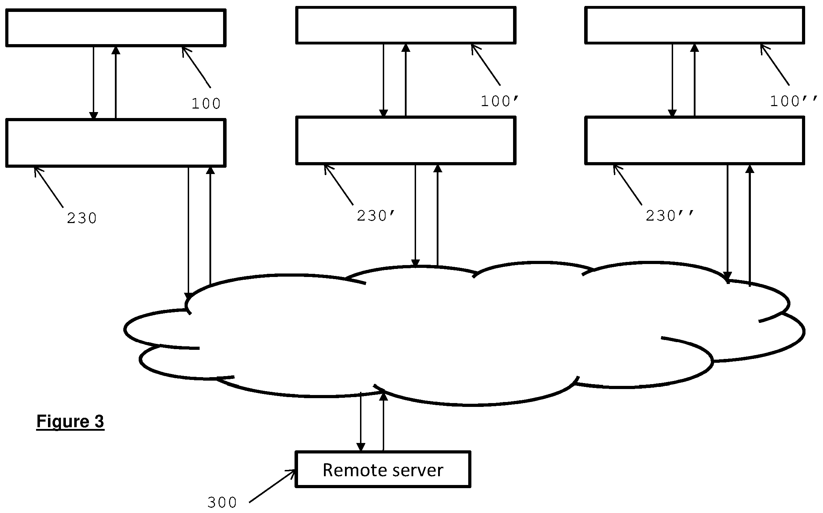

Figure 3 illustrates an exemplary embodiment with multiple prostheses or orthoses 100, 100', 100". Each prosthesis or orthosis 100, 100', 100" comprises a respective data storing means for storing data related to an operation of the respective prosthesis or orthosis 100, 100', 100" and/or to an environment of the respective prosthesis or orthosis 100, 100', 100". Each prosthesis or orthosis 100, 100', 100" is associated with a respective communication module 230, 230', 230" comprising a communication interface configured for transferring at least part of the stored data and/or processed data based thereon, to a remote server 300. The remote server 300 is configured for determining respective configuration and/or behavioral data for each prosthesis or orthosis 100, 100', 100", based on the transferred data received from the multiple prostheses or orthoses 100, 100', 100", and for transmitting the respective configuration and/or behavioral data to the respective communication module 230, 230', 230" associated with the respective prosthesis or orthosis 100, 100', 100".

More in particular, determining of the configuration and/or behavioral data for the first prosthesis or orthosis 100, may be based on the transferred data received from the first prosthesis or orthosis 100, but optionally also based on data received from one or more other prostheses or orthoses 100', 100". Similarly, determining of the configuration and/or behavioral data for the second prosthesis or orthosis 100', may be based on the transferred data received from the second prosthesis or orthosis 100', but optionally also based on data received from one or more other prostheses or orthoses 100, 100". In that way the configuration and/or behavioral data may be personalized for a particular user whilst at the same time taking into account problems encountered by another user.

The prosthesis or orthosis 100, 100', 100" may be implemented as described in any one of the embodiments above. The communication module 230, 230', 230" may be included in a separate device or may be included in the prosthesis or orthosis 100, 100', 100", as described in any one of the embodiments above.

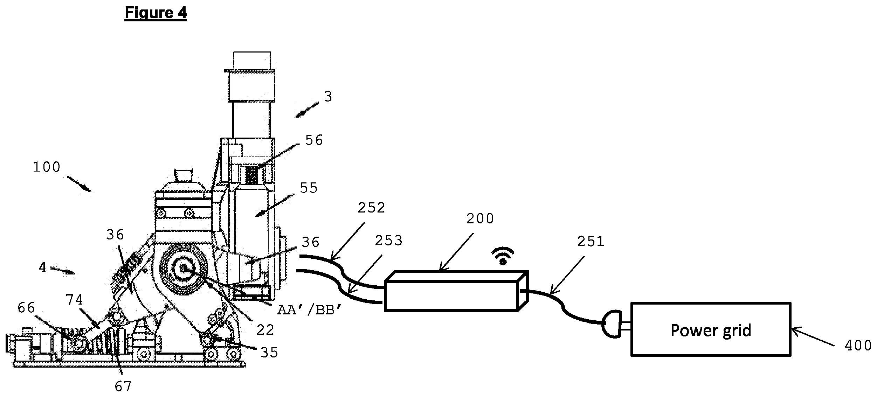

Figure 4 illustrated an exemplary embodiment of a system comprising a prosthesis 100 in a state removed form the wearer, and a separate device 200 connected to a power grid 400.

The prosthesis 100 comprises a hinge joint system and is intended for functionally replacing a hinge joint of a human subject. In this particular case the prosthesis 100 is a foot-ankle prosthesis for replacing the foot of a person in a transtibial or a transfemoral manner. The hinge joint system comprises a first member 3 and a second member 4 which are interconnected for a rotational movement in respect to one another. The second member 4 is forming an artificial foot replacing the missing foot of the concerned person. Two lever arms 35 and 36 pivote around the ankle axis BB'. The foot 4 is connected to lever arm 35 through a crank slider mechanism. On the foot part 4, and more precisely on the slider 66 of the crank-slider mechanism are the springs 67 which allows the prosthesis 100 to function as an efficient ESR foot in passive mode (when the actuator 55, see further, is not activated).

The prosthesis 100 comprises a force, resistance or motion generating device implemented as an actuator 55, e.g. a motor, put in series with an elastic element 56. The prosthesis 100 comprises further comprises a movement controlling mechanism comprising a clutch assembly 22. The actuator 55 (which corresponds with an example of the force, resistance or motion generating device 150 of Figure 2) provides mechanical energy for moving the one or more intermediate elements (which correspond with an example of the component 170 of Figure 2) of the movement controlling mechanism. The actuator 55 serves as a means by which, on the one hand, energy can be accumulated, so that a sufficiently high energy level can be reached in order to switch the movement controlling mechanism between a contacting mode of operation and a released mode of operation, and, on the other hand, the timing of this switch-over can be controlled.

The prosthesis 100 may have any one or more of the features disclosed in WO2016009308 which is included herein by reference.

The separate device 200 may be implemented as described above, e.g. in connection with Figure 2. The various components of the separate device 200 may be included in a housing provided with a number of connection interfaces, such as a connection interface 251 for connection the separate device 200 to the power grid 400, and a connection interface 252 for providing power to the prosthesis 100, and a connection interface 253 for exchanging data with the prosthesis 100. Connection interface 251 may be connected to connection interface 252 through a charging means 240 as described above in connection with Figure 2.

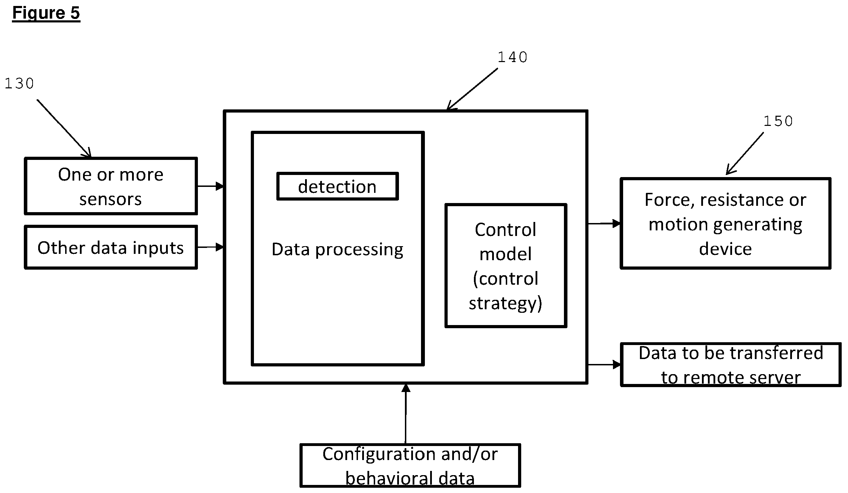

Figure 5 illustrates an example where the orthosis or prosthesis comprises a force, resistance or motion generating device 150, such as an actuator or a damper, for influencing the mechanical behavior and/or movement of a component (not shown) of the orthosis or prosthesis, and a control module 140 configured to control the device 150 according to a control model. The configuration and/or behavioral data received from the remote server may be used to modify the control model.

The control model 140 may receive one or more inputs and may use one or more weight parameters to generate one or more outputs based on the one or more inputs. The control model may define a control policy, e.g. as known in the technical domain of artificial intelligence. For example, the one or more inputs may comprise outputs of one or more sensors 130, and/or processed data based on the outputs of one or more sensors 130, and/or other data inputs. For example, the sensor data may further be processed in accordance with a detection algorithm to determine one or more operation categories of the prosthesis, e.g. an activity mode such as whether the person is walking on flat ground or on a slope, whether the person is standing, walking, or running, etc. These one or more operation categories may then be input in the control model. The control model may use one or more weight parameters associated with the different inputs. The one or more outputs of the control model allow the control module 140 to control the mechanical impact of the device 150. The weight parameters may be used for adaptive control and different sets of weights can be defined based on the activity mode of the prosthesis or orthosis and based on the movement achieved by the prosthesis or orthosis.

Optionally the control module 140 may also output data to be stored in a data storing means in order to be transferred to the remote device.

In an embodiment, the configuration and/or behavioral data may define one or more new sources of inputs for the control model, and/or a new control model itself, and/or one or more parameters used by the control model.

Whilst the principles of the invention have been set out above in connection with specific embodiments, it is to be understood that this description is merely made by way of example and not as a limitation of the scope of protection which is determined by the appended claims.

## Claims

1. A data transfer method for a prosthesis or orthosis (100), said prosthesis or orthosis (100) including a data storing means (120) and a processing means (110), the method comprising :
- obtaining data (S11) related to an operation of the prosthesis or orthosis and/or to an environment of the prosthesis or orthosis;
- storing the obtained data (S12) and/or processed data based on the obtained data in the data storing means (120);
**characterized in that** the method further comprises:
- transferring (S13), using a communication module (230) connected to the prosthesis or orthosis (100), at least part of the stored data and/or processed data based on the stored data, to a remote server (300).

2. The method of claim 1, further comprising receiving (S21), using the communication module (230), configuration and/or behavioral data from the remote server (300); wherein the configuration and/or behavioral data are obtained by the remote server based on the transferred data.

3. The method of claim 2, wherein the orthosis or prosthesis (100) comprises a force, resistance or motion generating device (150), such as an actuator or a damper, for influencing the mechanical behavior and/or movement of a component (170) of the orthosis or prosthesis, and a control module (140) configured to control the force, resistance or motion generating device (150) according to a control model; and wherein the method comprises using the configuration and/or behavioral data received from the remote server (300) to adapt the control model.

4. The method of any one of the previous claims, wherein the prosthesis or orthosis further comprises at least one sensor means (130), and wherein obtaining data (S11) related to the operation of the prosthesis or orthosis and/or to the environment of the prosthesis or orthosis comprises obtaining sensor data from the at least one sensor means (130).

5. The method of claims 4, wherein the control module (140) controls the force, resistance or motion regulating device (150) based on sensor data of the at least one sensor means (130) in accordance with the control model; and
preferably, wherein the at least one sensor means (130) comprises multiple sensors, and wherein one or more sensors of said multiple sensors (130) are selected to provide an input of the control model based on the configuration and/or behavioral data received from the remote server.

6. The method of claim 4 or 5, wherein the at least one sensor means (130) comprises a first set of sensor means and a second set of sensor means; and wherein, depending on an activity mode of the prosthesis or orthosis (100) in use and/or on environmental data and/or on location data, first processed data is determined based on sensor data from the first set of sensor means, and/or second processed data is determined based on sensor data from the second set of sensor means, and said first and/or second processed data is transferred to the remote server (300); and/or
wherein the at least one sensor means comprises a first set of sensor means and a second set of sensor means, wherein first processed data is determined based on sensor data from the first set of sensor means and associated with a first priority, and second processed data is determined based on sensor data from the second set of sensor means and associated with a second priority, wherein a timing of the transferring of the first and/or second processed data to the remote server is based on the associated priority level.

7. The method of any one of the previous claims, further comprising:
- processing, by the processing means (110) of the prosthesis or orthosis, data related to the operation of the prosthesis or orthosis and/or to the environment of the prosthesis or orthosis stored in the data storing means (120), and storing said processed data in the data storing means (120);
- wherein the step of transferring data (S13) to the remote server (300) comprises transferring, using the communication module (230), at least part of the processed data to the remote server (300).

8. The method according to any one of the previous claims, wherein the communication module (230) is included in a separate device, wherein the separate device (200) comprises a second data storing means (220) connected to the communication module (230); and wherein the step (S13) of transferring data to the remote server (300) comprises a first step of transferring at least part of the stored data and/or processed data based on the stored data to the second data storing means (220), followed by a second step of transferring data stored in the second data storing means (220) and/or processed data based thereon to the remote server (300);
optionally, wherein the separate device (200) comprises a second processing means (210) configured for processing the data stored in the second data storing means (220), and configured for storing the processed data in the second data storing means (220);
optionally, wherein the first step of transferring to the second data storing means (220) is performed at more frequent time intervals than the second step of transferring to the remote server (300); and
optionally, wherein the first step of transferring to the second data storing means (220) is performed during a substantially inactive state of the prosthesis or orthosis.

9. The method of any one of the previous claims, further comprising obtaining further data (S11) related to an operation of the prosthesis or orthosis and/or to an environment of the prosthesis or orthosis and determining whether or not the further obtained data (S12) is to be stored based on the stored data in the data storing means (120); and/or wherein an identification of the prosthesis or orthosis or of the communication module is added to the data which is transferred to the remote server.

10. The method of any one of the previous claims, wherein the prosthesis or orthosis (100) corresponds with a first prosthesis or orthosis, and wherein the method further comprises the following steps performed by a second prosthesis or orthosis (100'):
- obtaining second data related to an operation of the second prosthesis or orthosis and/or to an environment of the second prosthesis or orthosis;
- storing the obtained second data and/or processed data based on the obtained second data;
- transferring, using a communication module connected to the second prosthesis or orthosis, at least part of the stored second data and/or processed second data based on the stored second data, to the remote server;
and the following steps performed by the remote server:
- determining of first and second configuration and/or behavioral data for the first and second prosthesis or orthosis, respectively, based on the transferred data received from the first and second prosthesis or orthosis;
- transmitting the determined first and second configuration and/or behavioral data to the communication module associated with the first and second prosthesis or orthosis, respectively.

11. A data treatment method for a prosthesis or an orthosis, the method performed in a remote server comprising;
- receiving data (S21) from a communication module (230), said data comprising data related to an operation of the prosthesis or orthosis and/or to an environment of the prosthesis or orthosis and/or processed data based on said data related to the operation of the prosthesis or orthosis and/or to the environment of the prosthesis or orthosis;
- determining configuration and/or behavioral data (S22) for operating the prosthesis or orthosis (100) based on the received data;
- sending the configuration and/or behavioral data (S23) to the communication module (230).

12. The data treatment method of claim 11, wherein the prosthesis or orthosis (100) corresponds with a first prosthesis or orthosis, and wherein the method further comprises:
- receiving second data from a communication module associated with a second prosthesis or orthosis, said second data comprising data related to an operation of the second prosthesis or orthosis and/or to an environment of the prosthesis or orthosis and/or processed data based on said data;
- determining second configuration and/or behavioral data for the second prosthesis or orthosis, based on the received second data, and further based on the data received from the first prosthesis or orthosis;
- sending the second configuration and/or behavioral data to the communication module associated with the second prosthesis or orthosis.

13. A system comprising a prosthesis or orthosis (100) and a communication module (230), for use in a method of any one of the claims 1-10, said prosthesis or orthosis (100) comprising a data storing means (120) and a processing means (110),
wherein the data storing means (120) is configured for storing data related to an operation of the prosthesis or orthosis and/or to an environment of the prosthesis or orthosis,
wherein the communication module (230) comprises a communication interface configured for transferring at least part of the stored data and/or processed data based thereon, to a remote server (300); and
optionally, wherein the system is configured to add an identification of the prosthesis or orthosis or of the communication module to the data which is transferred to the remote server.

14. The system of claim 13, wherein the communication module (230) is configured to receive configuration and/or behavioral data from the remote server (300); wherein the remote server is configured for determining the configuration and/or behavioral data based on the transferred data;
optionally, wherein the orthosis or prosthesis (100) comprises a force, resistance or motion generating device (150), such as an actuator or a damper, for influencing the mechanical behavior and/or movement of a component (170) of the orthosis or prosthesis, and a control module (140) configured to control the force, resistance or motion generating device (150) according to a control model; and wherein the configuration and/or behavioral data received from the remote server (300) is used to adapt the control model; and
optionally, wherein the prosthesis or orthosis (100) further comprises at least one sensor means (130) for obtaining sensor data related to the operation of the prosthesis or orthosis and/or to the environment of the prosthesis or orthosis, and wherein the storing means is configured to store the sensor data.

15. The system of claims 13 or 14, wherein the communication module (230) is included in a separate device (200); and
preferably, wherein the separate device (200) is an energy charging device, and wherein the prosthesis or orthosis comprises an energy storage means (160); wherein the energy charging device is configured for charging the energy storage means (160); and
preferably, wherein the separate device (200) comprises a second data storing means (220) connected to the communication module (230), and optionally a second processing means (210) configured for processing data stored in the second data storing means (220), and configured for storing the processed data in the second data storing means (210).

## Patentansprüche

1. Datenübertragungsverfahren für eine Prothese oder Orthese (100), wobei die Prothese oder Orthese (100) ein Datenspeichermittel (120) und ein Verarbeitungsmittel (110) einschließt, das Verfahren umfassend:
- Erhalten von Daten (S11), die sich auf einen Betrieb der Prothese oder Orthese und/oder auf eine Umgebung der Prothese oder Orthese beziehen;
- Speichern der erhaltenen Daten (S12) und/oder verarbeiteten Daten basierend auf den erhaltenen Daten in dem Datenspeichermittel (120);
**dadurch gekennzeichnet, dass** das Verfahren ferner umfasst:
- Übertragen (S13), unter Verwendung Kommunikationsmoduls (230), das mit der Prothese oder Orthese (100) verbunden ist, mindestens eines Teils der gespeicherten Daten und/oder verarbeiteten Daten basierend auf den gespeicherten Daten an einen entfernten Server (300).

2. Verfahren nach Anspruch 1, ferner umfassend ein Empfangen (S21), unter Verwendung des Kommunikationsmoduls (230), von Konfigurations- und/oder Verhaltensdaten von dem entfernten Server (300); wobei die Konfigurations- und/oder Verhaltensdaten durch den entfernten Server basierend auf den übertragenen Daten erhalten werden.

3. Verfahren nach Anspruch 2, wobei die Orthese oder Prothese (100) eine Kraft-, Widerstands- oder Bewegungsgenerierungsvorrichtung (150), wie einen Aktuator oder einen Dämpfer, zum Beeinflussen des mechanischen Verhaltens und/oder der Bewegung einer Komponente (170) der Orthese oder Prothese und ein Steuermodul (140) umfasst, das konfiguriert ist, um die Kraft-, Widerstands- oder Bewegungsgenerierungsvorrichtung (150) gemäß einem Steuermodell zu steuern; und wobei das Verfahren das Verwenden der Konfigurations- und/oder Verhaltensdaten umfasst, die von dem entfernten Server (300) empfangen werden, um das Steuerungsmodell anzupassen.

4. Verfahren nach einem der vorstehenden Ansprüche, wobei die Prothese oder Orthese ferner mindestens ein Sensormittel (130) umfasst, und wobei das Erhalten von Daten (S11), die sich auf den Betrieb der Prothese oder Orthese und/oder auf die Umgebung der Prothese oder Orthese beziehen, das Erhalten von Sensordaten von dem mindestens einen Sensormittel (130) umfasst.

5. Verfahren nach Anspruch 4, wobei das Steuermodul (140) die Kraft-, Widerstands- oder Bewegungsgenerierungsvorrichtung (150) basierend auf Sensordaten des mindestens einen Sensormittels (130) gemäß dem Steuermodell steuert; und
vorzugsweise, wobei das mindestens eine Sensormittel (130) mehrere Sensoren umfasst, und wobei ein oder mehrere Sensoren der mehreren Sensoren (130) ausgewählt sind, um eine Eingabe des Steuerungsmodells basierend auf den Konfigurations- und/oder Verhaltensdaten bereitzustellen, die von dem entfernten Server empfangen werden.

6. Verfahren nach Anspruch 4 oder 5, wobei das mindestens eine Sensormittel (130) einen ersten Satz von Sensormitteln und einen zweiten Satz von Sensormitteln umfasst; und wobei, abhängig von einem Aktivitätsmodus der Prothese oder Orthese (100) in Verwendung und/oder von Umgebungsdaten und/oder von Standortdaten, erste verarbeitete Daten basierend auf Sensordaten aus dem ersten Satz von Sensormitteln bestimmt werden, und/oder zweite verarbeitete Daten basierend auf Sensordaten aus dem zweiten Satz von Sensormitteln bestimmt werden, und die ersten und/oder die zweiten verarbeiteten Daten an den entfernten Server (300) übertragen werden; und/oder
wobei das mindestens eine Sensormittel einen ersten Satz von Sensormitteln und einen zweiten Satz von Sensormitteln umfasst, wobei erste verarbeitete Daten basierend auf Sensordaten aus dem ersten Satz von Sensormitteln bestimmt werden und einer ersten Priorität zugeordnet sind, und zweite verarbeitete Daten basierend auf Sensordaten aus dem zweiten Satz von Sensormitteln bestimmt werden und einer zweiten Priorität zugeordnet sind, wobei eine Zeitsteuerung des Übertragens der ersten und/oder der zweiten verarbeiteten Daten an den entfernten Server auf der zugeordneten Prioritätsstufe basiert.

7. Verfahren nach einem der vorstehenden Ansprüche, ferner umfassend:
- Verarbeiten, durch das Verarbeitungsmittel (110) der Prothese oder Orthese, von Daten, die sich auf den Betrieb der Prothese oder Orthese und/oder auf die Umgebung der Prothese oder Orthese beziehen, die in dem Datenspeichermittel (120) gespeichert sind, und Speichern der verarbeiteten Daten in dem Datenspeichermittel (120);
- wobei der Schritt des Übertragens von Daten (S13) an den entfernten Server (300) das Übertragen, unter Verwendung des Kommunikationsmoduls (230), von mindestens einem Teil der verarbeiteten Daten an den entfernten Server (300) umfasst.

8. Verfahren nach einem der vorstehenden Ansprüche, wobei das Kommunikationsmodul (230) in einer separaten Vorrichtung eingeschlossen ist, wobei die separate Vorrichtung (200) ein zweites Datenspeichermittel (220) umfasst, das mit dem Kommunikationsmodul (230) verbunden ist; und wobei der Schritt (S13) des Übertragens von Daten an den entfernten Server (300) einen ersten Schritt des Übertragens mindestens eines Teils der gespeicherten Daten und/oder verarbeiteten Daten basierend auf den gespeicherten Daten an das zweite Datenspeichermittel (220) umfasst, gefolgt von einem zweiten Schritt des Übertragens von Daten, die in dem zweiten Datenspeichermittel (220) gespeichert sind, und/oder verarbeiteten Daten, die darauf basieren, an den entfernten Server (300);
optional, wobei die separate Vorrichtung (200) ein zweites Verarbeitungsmittel (210) umfasst, das zum Verarbeiten der Daten konfiguriert ist, die in dem zweiten Datenspeichermittel (220) gespeichert sind, und zum Speichern der verarbeiteten Daten in dem zweiten Datenspeichermittel (220) konfiguriert ist;
optional, wobei der erste Schritt des Übertragens an das zweite Datenspeichermittel (220) in häufigeren Zeitintervallen als der zweite Schritt des Übertragens an den entfernten Server (300) durchgeführt wird; und
optional, wobei der erste Schritt des Übertragens an das zweite Datenspeichermittel (220) während eines im Wesentlichen inaktiven Zustands der Prothese oder Orthese durchgeführt wird.

9. Verfahren nach einem der vorstehenden Ansprüche, ferner umfassend das Erhalten weiterer Daten (S11) bezogen auf einen Betrieb der Prothese oder Orthese und/oder auf eine Umgebung der Prothese oder Orthese und das Bestimmen, ob die weiteren erhaltenen Daten (S12) basierend auf den Daten zu speichern sind oder nicht, die in dem Datenspeichermittel (120) gespeichert sind; und/oder
wobei eine Identifikation der Prothese oder Orthese oder des Kommunikationsmoduls zu den Daten hinzugefügt wird, die an den entfernten Server übertragen werden.

10. Verfahren nach einem der vorstehenden Ansprüche, wobei die Prothese oder Orthese (100) einer ersten Prothese oder Orthese entspricht, und wobei das Verfahren ferner die folgenden Schritte umfasst, die durch eine zweite Prothese oder Orthese (100') durchgeführt werden:
- Erhalten von zweiten Daten, die sich auf einen Betrieb der zweiten Prothese oder Orthese und/oder auf eine Umgebung der zweiten Prothese oder Orthese beziehen;
- Speichern der erhaltenen zweiten Daten und/oder verarbeiteten Daten basierend auf den erhaltenen zweiten Daten;
- Übertragen, unter Verwendung eines Kommunikationsmoduls, das mit der zweiten Prothese oder Orthese verbunden ist, mindestens eines Teils der gespeicherten zweiten Daten und/oder verarbeiteten zweiten Daten basierend auf den gespeicherten zweiten Daten, an den entfernten Server;
und die folgenden Schritte, die durch den entfernten Server durchgeführt werden:
- Bestimmen von ersten und zweiten Konfigurations- und/oder Verhaltensdaten für die erste beziehungsweise die zweite Prothese oder Orthese, basierend auf den übertragenen Daten, die von der ersten und der zweiten Prothese oder Orthese empfangen werden;
- Übertragen der bestimmten ersten und zweiten Konfigurations- und/oder Verhaltensdaten an das Kommunikationsmodul, das der ersten beziehungsweise der zweiten Prothese oder Orthese zugeordnet ist.

11. Datenbehandlungsverfahren für eine Prothese oder eine Orthese, wobei das Verfahren in einem entfernten Server durchgeführt wird, umfassend;
- Empfangen von Daten (S21) von einem Kommunikationsmodul (230), die Daten umfassend Daten, die sich auf einen Betrieb der Prothese oder Orthese und/oder auf eine Umgebung der Prothese oder Orthese beziehen, und/oder verarbeitete Daten basierend auf den Daten, die sich auf den Betrieb der Prothese oder Orthese und/oder auf die Umgebung der Prothese oder Orthese beziehen;
- Bestimmen von Konfigurations- und/oder Verhaltensdaten (S22) zum Betreiben der Prothese oder Orthese (100) basierend auf den empfangenen Daten;
- Senden der Konfigurations- und/oder Verhaltensdaten (S23) an das Kommunikationsmodul (230).

12. Datenbehandlungsverfahren nach Anspruch 11, wobei die Prothese oder Orthese (100) einer ersten Prothese oder Orthese entspricht, und wobei das Verfahren ferner umfasst:
- Empfangen von zweiten Daten von einem Kommunikationsmodul, das einer zweiten Prothese oder Orthese zugeordnet ist, die zweiten Daten umfassend Daten, die sich auf einen Betrieb der zweiten Prothese oder Orthese und/oder auf eine Umgebung der Prothese oder Orthese beziehen und/oder verarbeitete Daten basierend auf den Daten;
- Bestimmen von zweiten Konfigurations- und/oder Verhaltensdaten für die zweite Prothese oder Orthese, basierend auf den empfangenen zweiten Daten und ferner basierend auf den Daten, die von der ersten Prothese oder Orthese empfangen werden;
- Senden der zweiten Konfigurations- und/oder Verhaltensdaten an das Kommunikationsmodul, das der zweiten Prothese oder Orthese zugeordnet ist.

13. System, umfassend eine Prothese oder Orthese (100) und ein Kommunikationsmodul (230), zur Verwendung in einem Verfahren nach einem der Ansprüche 1 bis 10, die Prothese oder Orthese (100) umfassend ein Datenspeichermittel (120) und ein Verarbeitungsmittel (110),
wobei das Datenspeichermittel (120) zum Speichern von Daten konfiguriert ist, die sich auf einen Betrieb der Prothese oder Orthese und/oder auf eine Umgebung der Prothese oder Orthese beziehen,
wobei das Kommunikationsmodul (230) eine Kommunikationsschnittstelle umfasst, die zum Übertragen, mindestens eines Teils der gespeicherten Daten und/oder verarbeiteten Daten, die darauf basieren, an einen entfernten Server (300) konfiguriert ist; und
optional, wobei das System konfiguriert ist, um eine Identifikation der Prothese oder Orthese oder des Kommunikationsmoduls zu den Daten hinzuzufügen, die an den entfernten Server übertragen werden.

14. System nach Anspruch 13, wobei das Kommunikationsmodul (230) konfiguriert ist, um Konfigurations- und/oder Verhaltensdaten von dem entfernten Server (300) zu empfangen; wobei der entfernte Server zum Bestimmen der Konfigurations- und/oder Verhaltensdaten basierend auf den übertragenen Daten konfiguriert ist;
optional, wobei die Orthese oder Prothese (100) eine Kraft-, Widerstands- oder Bewegungsgenerierungsvorrichtung (150), wie einen Aktuator oder einen Dämpfer, zum Beeinflussen des mechanischen Verhaltens und/oder der Bewegung einer Komponente (170) der Orthese oder Prothese, und ein Steuermodul (140) umfasst, das konfiguriert ist, um die Kraft-, Widerstands- oder Bewegungsgenerierungsvorrichtung (150) gemäß einem Steuermodell zu steuern; und wobei die Konfigurations- und/oder Verhaltensdaten, die von dem entfernten Server (300) empfangen werden, verwendet werden, um das Steuerungsmodell anzupassen; und
optional, wobei die Prothese oder Orthese (100) ferner mindestens ein Sensormittel (130) zum Erhalten von Sensordaten umfasst, die sich auf den Betrieb der Prothese oder Orthese und/oder auf die Umgebung der Prothese oder Orthese beziehen, und wobei das Speichermittel konfiguriert ist, um die Sensordaten zu speichern.

15. System nach Anspruch 13 oder 14, wobei das Kommunikationsmodul (230) in einer separaten Vorrichtung (200) eingeschlossen ist; und
vorzugsweise, wobei die separate Vorrichtung (200) eine Energieladevorrichtung ist, und wobei die Prothese oder Orthese ein Energiespeichermittel (160) umfasst; wobei die Energieladevorrichtung zum Laden des Energiespeichermittels (160) konfiguriert ist; und
vorzugsweise, wobei die separate Vorrichtung (200) ein zweites Datenspeichermittel (220), das mit dem Kommunikationsmodul (230) verbunden ist, und optional ein zweites Verarbeitungsmittel (210) umfasst, das zum Verarbeiten von Daten konfiguriert ist, die in dem zweiten Datenspeichermittel (220) gespeichert sind, und zum Speichern der verarbeiteten Daten in dem zweiten Datenspeichermittel (210) konfiguriert ist.

## Revendications

1. Procédé de transfert de données pour une prothèse ou orthèse (100), ladite prothèse ou orthèse (100) comportant un moyen de stockage de données (120) et un moyen de traitement (110), le procédé comprenant :
- l'obtention de données (S11) se rapportant à un fonctionnement de la prothèse ou orthèse et/ou à un environnement de la prothèse ou orthèse ;
- le stockage des données obtenues (S12) et/ou de données traitées en fonction des données obtenues dans le moyen de stockage de données (120) ;
**caractérisé en ce que** le procédé comprend en outre :
- le transfert (S13), à l'aide d'un module de communication (230) connecté à la prothèse ou orthèse (100), d'au moins une partie des données stockées et/ou des données traitées en fonction des données stockées, à un serveur distant (300).

2. Procédé selon la revendication 1, comprenant en outre la réception (S21), à l'aide du module de communication (230), de données de configuration et/ou de comportement à partir du serveur distant (300) ; dans lequel les données de configuration et/ou de comportement sont obtenues par le serveur distant en fonction des données transférées.

3. Procédé selon la revendication 2, dans lequel l'orthèse ou prothèse (100) comprend un dispositif de génération de force, de résistance ou de mouvement (150), tel qu'un actionneur ou un amortisseur, pour influencer le comportement et/ou mouvement mécanique d'un composant (170) de l'orthèse ou prothèse, et un module de commande (140) configuré pour commander le dispositif de génération de force, de résistance ou de mouvement (150) selon un modèle de commande ; et dans lequel le procédé comprend l'utilisation des données de configuration et/ou de comportement reçues à partir du serveur distant (300) pour adapter le modèle de commande.

4. Procédé selon l'une quelconque des revendications précédentes, dans lequel la prothèse ou orthèse comprend en outre au moins un moyen capteur (130), et dans lequel l'obtention de données (S11) se rapportant au fonctionnement de la prothèse ou orthèse et/ou à l'environnement de la prothèse ou orthèse comprend l'obtention de données de capteur à partir de l'au moins un moyen capteur (130).

5. Procédé selon la revendication 4, dans lequel le module de commande (140) commande le dispositif de génération de force, de résistance ou de mouvement (150) en fonction de données de capteur de l'au moins un moyen capteur (130) conformément au modèle de commande ; et
de préférence, dans lequel l'au moins un moyen capteur (130) comprend de multiples capteurs, et dans lequel un ou plusieurs capteurs desdits multiples capteurs (130) sont choisis pour fournir une entrée du modèle de commande en fonction des données de configuration et/ou de comportement reçues à partir du serveur distant.

6. Procédé selon la revendication 4 ou 5, dans lequel l'au moins **un** moyen capteur (130) comprend un premier ensemble de moyens capteurs et un second ensemble de moyens capteurs ; et dans lequel, en dépendance d'un mode d'activité de la prothèse ou orthèse (100) en cours d'utilisation et/ou de données environnementales et/ou de données de localisation, des premières données traitées sont déterminées en fonction de données de capteur à partir du premier ensemble de moyens capteurs, et/ou des secondes données traitées sont déterminées en fonction de données de capteur à partir du second ensemble de moyens capteurs, et lesdites premières et/ou secondes données traitées sont transférées au serveur distant (300) ; et/ou
dans lequel l'au moins un moyen capteur comprend un premier ensemble de moyens capteurs et un second ensemble de moyens capteurs, dans lequel des premières données traitées sont déterminées en fonction de données de capteur à partir du premier ensemble de moyens capteurs et associées à une première priorité, et des secondes données traitées sont déterminées en fonction de données de capteur à partir du second ensemble de moyens capteurs et associées à une seconde priorité, dans lequel un moment du transfert des premières et/ou secondes données traitées au serveur distant est en fonction du niveau de priorité associé.

7. Procédé selon l'une quelconque des revendications précédentes, comprenant en outre :
- le traitement, par le moyen de traitement (110) de la prothèse ou orthèse, de données se rapportant au fonctionnement de la prothèse ou orthèse et/ou à l'environnement de la prothèse ou orthèse stockées dans le moyen de stockage de données (120), et le stockage desdites données traitées dans le moyen de stockage de données (120) ;
- dans lequel l'étape de transfert de données (S13) au serveur distant (300) comprend le transfert, à l'aide du module de communication (230), d'au moins une partie des données traitées au serveur distant (300).

8. Procédé selon l'une quelconque des revendications précédentes, dans lequel le module de communication (230) est intégré dans un dispositif séparé, dans lequel le dispositif séparé (200) comprend un second moyen de stockage de données (220) connecté au module de communication (230) ; et dans lequel l'étape (S13) de transfert de données au serveur distant (300) comprend une première étape de transfert d'au moins une partie des données stockées et/ou des données traitées en fonction des données stockées au second moyen de stockage de données (220), suivie d'une seconde étape de transfert de données stockées dans le second moyen de stockage de données (220) et/ou de données traitées en fonction de celles-ci au serveur distant (300) ;
éventuellement, dans lequel le dispositif séparé (200) comprend un second moyen de traitement (210) configuré pour traiter les données stockées dans le second moyen de stockage de données (220), et configuré pour stocker les données traitées dans le second moyen de stockage de données (220) ;
éventuellement, dans lequel la première étape de transfert au second moyen de stockage de données (220) est réalisée à des intervalles de temps plus fréquents que la seconde étape de transfert au serveur distant (300) ; et
éventuellement, dans lequel la première étape de transfert au second moyen de stockage de données (220) est réalisée pendant un état sensiblement inactif de la prothèse ou orthèse.

9. Procédé selon l'une quelconque des revendications précédentes, comprenant en outre l'obtention d'autres données (S11) se rapportant à un fonctionnement de la prothèse ou orthèse et/ou à un environnement de la prothèse ou orthèse et la détermination de si les autres données (S12) obtenues doivent ou non être stockées en fonction des données stockées dans le moyen de stockage de données (120) ; et/ou
dans lequel une identification de la prothèse ou orthèse ou du module de communication est ajoutée aux données qui sont transférées au serveur distant.

10. Procédé selon l'une quelconque des revendications précédentes, dans lequel la prothèse ou orthèse (100) correspond à une première prothèse ou orthèse, et dans lequel le procédé comprend en outre les étapes suivantes réalisées par une seconde prothèse ou orthèse (100') :
- l'obtention de secondes données se rapportant à un fonctionnement de la seconde prothèse ou orthèse et/ou à un environnement de la seconde prothèse ou orthèse ;
- le stockage des secondes données obtenues et/ou des données traitées en fonction des secondes données obtenues ;
- le transfert, à l'aide d'un module de communication connecté à la seconde prothèse ou orthèse, d'au moins une partie des secondes données stockées et/ou des secondes données traitées en fonction des secondes données stockées, au serveur distant ;
et les étapes suivantes réalisées par le serveur distant :
- la détermination de premières et de secondes données de configuration et/ou de comportement pour la première et la seconde prothèse ou orthèse, respectivement, en fonction des données transférées reçues à partir de la première et de la seconde prothèse ou orthèse ;
- la transmission des premières et secondes données de configuration et/ou de comportement déterminées au module de communication associé à la première et à la seconde prothèse ou orthèse, respectivement.

11. Procédé de traitement de données pour une prothèse ou une orthèse, le procédé étant réalisé dans un serveur distant comprenant ;
- la réception de données (S21) à partir d'un module de communication (230), lesdites données comprenant des données se rapportant à un fonctionnement de la prothèse ou orthèse et/ou à un environnement de la prothèse ou orthèse et/ou des données traitées en fonction desdites données se rapportant au fonctionnement de la prothèse ou orthèse et/ou à l'environnement de la prothèse ou orthèse ;
- la détermination de données de configuration et/ou de comportement (S22) pour le fonctionnement de la prothèse ou orthèse (100) en fonction des données reçues ;
- l'envoi des données de configuration et/ou de comportement (S23) au module de communication (230).

12. Procédé de traitement de données selon la revendication 11, dans lequel la prothèse ou orthèse (100) correspond à une première prothèse ou orthèse, et dans lequel le procédé comprend en outre :
- la réception de secondes données à partir d'un module de communication associé à une seconde prothèse ou orthèse, lesdites secondes données comprenant des données se rapportant à un fonctionnement de la seconde prothèse ou orthèse et/ou à un environnement de la prothèse ou orthèse et/ou des données traitées en fonction desdites données ;
- la détermination de secondes données de configuration et/ou de comportement pour la seconde prothèse ou orthèse, en fonction des secondes données reçues, et en outre en fonction des données reçues à partir de la première prothèse ou orthèse ;
- l'envoi des secondes données de configuration et/ou de comportement au module de communication associé à la seconde prothèse ou orthèse.

13. Système comprenant une prothèse ou orthèse (100) et un module de communication (230), destiné à être utilisé dans un procédé selon l'une quelconque des revendications 1 à 10, ladite prothèse ou orthèse (100) comprenant un moyen de stockage de données (120) et un moyen de traitement (110),
dans lequel le moyen de stockage de données (120) est configuré pour stocker des données se rapportant à un fonctionnement de la prothèse ou orthèse et/ou à un environnement de la prothèse ou orthèse,
dans lequel le module de communication (230) comprend une interface de communication configurée pour transférer au moins une partie des données stockées et/ou des données traitées en fonction de celles-ci, à un serveur distant (300) ; et
éventuellement, dans lequel le système est configuré pour ajouter une identification de la prothèse ou orthèse ou du module de communication aux données qui sont transférées au serveur distant.

14. Système selon la revendication 13, dans lequel le module de communication (230) est configuré pour recevoir des données de configuration et/ou de comportement à partir du serveur distant (300) ; dans lequel le serveur distant est configuré pour déterminer les données de configuration et/ou de comportement en fonction des données transférées ;
éventuellement, dans lequel l'orthèse ou prothèse (100) comprend un dispositif de génération de force, de résistance ou de mouvement (150), tel qu'un actionneur ou un amortisseur, pour influencer le comportement et/ou mouvement mécanique d'un composant (170) de l'orthèse ou prothèse, et un module de commande (140) configuré pour commander le dispositif de génération de force, de résistance ou de mouvement (150) selon un modèle de commande ; et dans lequel les données de configuration et/ou de comportement reçues à partir du serveur distant (300) sont utilisées pour adapter le modèle de commande ; et
éventuellement, dans lequel la prothèse ou orthèse (100) comprend en outre au moins un moyen capteur (130) pour obtenir des données de capteur se rapportant au fonctionnement de la prothèse ou orthèse et/ou à l'environnement de la prothèse ou orthèse, et dans lequel le moyen de stockage est configuré pour stocker les données de capteur.

15. Système selon les revendications 13 ou 14, dans lequel le module de communication (230) est intégré dans un dispositif séparé (200) ; et
de préférence, dans lequel le dispositif séparé (200) est un dispositif de charge d'énergie, et dans lequel la prothèse ou orthèse comprend un moyen de stockage d'énergie (160) ; dans lequel le dispositif de charge d'énergie est configuré pour charger le moyen de stockage d'énergie (160) ; et
de préférence, dans lequel le dispositif séparé (200) comprend un second moyen de stockage de données (220) connecté au module de communication (230), et éventuellement un second moyen de traitement (210) configuré pour traiter des données stockées dans le second moyen de stockage de données (220), et configuré pour stocker les données traitées dans le second moyen de stockage de données (210).
